# FASCICULE DE BREVET EUROPEEN

(11) **EP 1 477 906 B1**
(45) Date de publication et mention de la délivrance du brevet: **17.10.2018**
(21) Numéro de dépôt: 04291236.0
(22) Date de dépôt: 14.05.2004
(51) Int. Cl.: G06F 17/00, G06F 11/10

(54) **Dispositif médical actif pourvu d'une mémoire pour le stockage de données Holter et d'instructions de pilotage d'un microprocesseur**
Aktive medizinische Vorrichtung mit einem Speicher zur Ablage von Holter-Daten und einem Steuerprogramm für einen Mikroprozessor
Active medical apparatus equipped with a memory for storing holter data and a microprocessor control program

(30) Priorité: 14.05.2003 FR 0305751
(43) Date de publication de la demande: 17.11.2004
(73) Titulaire: ELA MEDICAL, 92541 Montrouge (FR)
(72) Inventeur: Legay, Thierry, 91640 Fontenay Les Briis (FR); Amara, Karima, 92330 Sceaux (FR)
(74) Mandataire: Grünecker Patent- und Rechtsanwälte PartG mbB

(56) Documents cités:
- US-A- 5 669 393
- US-A1- 2003 070 054
- US-B1- 6 185 696

## Description

L'invention concerne les "dispositifs médicaux actifs", notamment les "dispositifs médicaux implantables actifs" tels que définis par la Directive 90/385/CE du 20 juin 1990 du Conseil des communautés européennes. Cette définition inclut les stimulateurs cardiaques, défibrillateurs, cardioverteurs et/ou dispositifs multisite, mais aussi les appareils neurologiques, les pompes de diffusion de substances médicales, les implants cochléaires, les capteurs biologiques implantés, etc., ainsi que les dispositifs de mesure de pH ou encore de mesure d'impédance intracorporelle (telle que la mesure de l'impédance transpulmonaire ou de l'impédance intracardiaque).

L'invention s'applique de manière particulièrement avantageuse à ceux de ces dispositifs qui mettent en oeuvre des fonctions d'enregistrement de données, appelées "données Holter", enregistrées sur une longue période, de plusieurs jours à plusieurs mois, principalement relatives à l'activité cardiaque mais pouvant aussi inclure des compteurs d'événements ou des signaux non recueillis mais représentatifs d'un état ou d'une action de l'implant, par exemple l'application d'une thérapie de choc, la mesure d'une impédance de sonde, etc.

Dans la présente description on se référera principalement aux appareils implantés tels que stimulateurs, cardioverteurs ou défibrillateurs, mais l'invention peut être également mise en oeuvre avec des appareils Holter simples, externes, ayant pour seule fonction la surveillance et l'enregistrement en ambulatoire de l'activité cardiaque.

Un tel appareil Holter externe est par exemple décrit dans US 5,669,393 A.

L'architecture habituelle des circuits électroniques d'un stimulateur ou défibrillateur cardiaque implantable comprend une unité centrale, avec une mémoire de type ROM (mémoire morte) intégrée au microcontrôleur et contenant le code logiciel permettant de piloter la prothèse cardiaque, ainsi qu'une RAM (mémoire vive) de grande capacité pour le stockage des données recueillies par la prothèse. Le circuit comporte également des éléments d'interfaçage avec les circuits analogiques, ainsi qu'éventuellement un circuit DMA (accès direct à la mémoire) et divers composants périphériques nécessaires à la mise en oeuvre de la prothèse.

Une première difficulté, avec ce type d'architecture classique, réside dans la difficulté de mise à jour du code logiciel. En effet, pour mettre à jour ce code une opération dite de "remasquage" du microcontrôleur ou microprocesseur est nécessaire. Cette opération est prise en charge par le fabricant du circuit (le fondeur) et nécessite une durée longue, typiquement de deux à trois mois. Lorsque le nouveau circuit est livré, il faut ensuite fabriquer les prothèses l'incorporant, ce qui ajoute encore deux à trois mois supplémentaires correspondant à la durée complète d'un cycle de fabrication industrielle. Ainsi, le délai entre l'élaboration d'une nouvelle version du code logiciel et son introduction commerciale dans des produits livrables au praticien est en général d'au moins six mois.

Un premier but de l'invention est de pallier cet inconvénient, en offrant un gain de souplesse et de temps de cycle pour la mise à jour du code logiciel, grâce à un stockage de ce logiciel dans un circuit RAM et non plus ROM, mais avec une protection adéquate, car un circuit RAM ne présente pas les mêmes garanties de sûreté qu'un circuit ROM : avec un circuit ROM, l'intégrité de l'information est garantie en tout état de cause, tandis que l'information conservée dans un circuit RAM peut être altérée notamment par des rayonnements tels que les particules alpha, qui peuvent modifier de façon aléatoire l'état d'un bit d'information (altération dite *Single Event Upset* ou SEU).

Un autre but de l'invention est de réduire le nombre de puces nécessaires à la réalisation d'une prothèse cardiaque implantable.

Comme on le sait, la miniaturisation est un facteur essentiel pour ce type de dispositif, et une architecture permettant de gagner de la surface sur le circuit hybride portant les différentes puces, en diminuant le nombre de ces puces, constitue un facteur de progrès important. L'un des buts de l'invention est ainsi de proposer une architecture qui permette de n'utiliser qu'un seul circuit RAM pour stocker à la fois le code logiciel (avec l'avantage corrélatif indiqué plus haut d'une mise à jour aisée) et les données Holter.

Par ailleurs, on sait que les techniques Holter requièrent des ressources mémoire importantes si l'on veut disposer de données à la fois précises et couvrant une période étendue. Ainsi, on souhaite aujourd'hui pouvoir étendre le suivi du patient sur une durée de six mois avec une résolution temporelle d'un jour ou moins, tout en enregistrant un volume important de données et d'électrogrammes (EGM) recueillis juste avant un évènement indésirable ("stockage d'EGM pré-événementiel"), et après traitement de cet événement par la prothèse, par exemple après application d'un choc de défibrillation ou de cardioversion, ou après une modification du mode de stimulation ("stockage post-évènementiel").

C'est pourquoi un autre but encore de l'invention est de proposer une architecture qui permette de stocker à la fois le code logiciel et les données Holter avec des capacités d'adressage étendues, gérées indépendamment pour ces deux types d'informations de manière à maximiser le volume de données Holter stockées.

En d'autres termes, l'invention a pour objet une architecture permettant, à surface minimale sur un même circuit RAM, de stocker de grandes quantités mémoires de données Holter d'une part, et de stocker en toute sécurité tout ou partie du code logiciel, d'autre part.

Pour atteindre les divers buts précités, l'invention propose un dispositif médical actif comportant, de manière en elle-même connue d'après le document US 5,669,393 A précité, les éléments énoncés au préambule de la revendication 1. Les éléments propres à l'invention sont énoncés dans la partie caractérisante de la revendication 1.

Les sous-revendications énoncent des formes de mise en oeuvre avantageuses de l'invention.

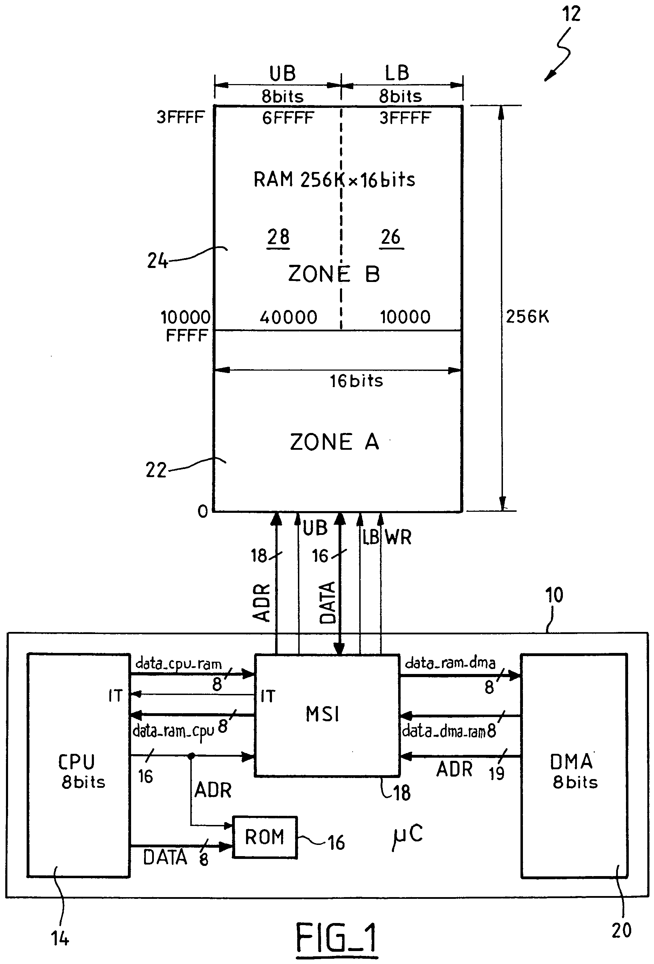

On va maintenant décrire un exemple de mise en oeuvre du dispositif de l'invention en référence au dessin annexé, où la figure unique est un schéma par blocs fonctionnels illustrant les différents circuits mis en oeuvre par l'invention et la manière dont ils interagissent.

Sur la figure, la référence 10 désigne le microcontrôleur du dispositif implantable, qui coopère avec une mémoire externe 12 qui est un circuit RAM de grande capacité intégré dans une puce distincte du microcontrôleur. Cette mémoire 12 peut être un circuit d'un type classique, fonctionnant en basse tension (1,8 V ou moins) avec une capacité de 128, 256 ou 512 K octets, capacité permettant de mémoriser quelques dizaines de minutes d'enregistrement EGM, comme requis par les techniques de diagnostic les plus récentes. Dans l'exemple illustré, la RAM est une RAM de 512 K octets, organisée physiquement en 256 K mots de 16 bits.

Le microcontrôleur 10 comprend une unité centrale de traitement (CPU) 14, qui dans l'exemple illustré est un circuit dit "1 octet", c'est-à-dire travaillant sur 8 bits en parallèle. Cette unité centrale 14 est interfacée avec une mémoire morte (circuit ROM) 16 contenant le code d'exploitation du processeur de l'unité 14, ainsi que différents éléments de code logiciel propres à l'invention et que l'on exposera plus bas. Cette mémoire morte 16 est organisée en mots de 8 bits, adressables sur 16 bits.

Le microcontrôleur 10 comprend également un module spécifique d'interface (MSI) 18, propre à l'invention, dont on décrira en détail les fonctions ci-après et dont le rôle général est d'organiser la communication entre l'unité centrale 14 et la mémoire RAM externe 12.

Le microcontrôleur 10 peut également comprendre un circuit d'accès direct mémoire (DMA) 20, interfacé également avec la mémoire RAM 12 par le MSI 18.

L'unité centrale 14 possède un bus de données sur 8 bits pour la lecture de données en RAM (bus data_ram_cpu), et pour l'écriture de données en RAM (bus data_cpu_ram). Il en est de même pour le circuit DMA 20 éventuellement présent (bus data_ram_dma et data_dma_ram).

De façon caractéristique de l'invention, la mémoire RAM 12 est utilisée à la fois :
- pour le stockage du code logiciel, dans une première zone (zone A, référencée 22), avec une protection logique gérée par le MSI, et
- pour le stockage des données Holter, dans une deuxième zone (zone B, référencée 24), sans protection logique.

La mémoire 12 est ainsi partagée en deux zones principales, A et B.

La zone A, réservée au code logiciel du programme permettant de faire fonctionner l'unité centrale 14, est organisée en mots de 16 bits, de l'adresse 0 à l'adresse FFFF (adresses physiques et logiques).

Chaque mot stocké dans cette zone comprend 8 bits de code opération, auquel est adjoint un code détecteur et correcteur (CDCE) de 1 à 8 bits.

La nature du CDCE est variable et choisie parmi les codes de protection classiques utilisés dans l'industrie tels que contrôle de parité, *checksum,* code de redondance cyclique (CRC), etc. Suivant la performance recherchée dans la détection et la correction d'erreur, on peut utiliser de 1 à 8 bits pour stocker ce CDCE. L'utilisation d'un nombre élevé de bits accroît la performance du CDCE, mais présente l'inconvénient d'augmenter la consommation car, pour un même nombre de lignes (16 lignes), le bus de données comprendra un nombre plus élevé de lignes actives, avec donc des pertes plus élevées dans les capacités parasites du substrat ; de même, le MSI consommera plus d'énergie car la gestion du CDCE sera plus complexe.

Or la consommation du microcalculateur est un facteur qui doit être soigneusement maîtrisé, car elle a une incidence directe sur la durée de vie de l'implant, c'est-à-dire le nombre d'années au bout duquel une intervention sera nécessaire pour échanger l'implant, arrivé en fin de vie.

Un compromis satisfaisant consiste généralement à utiliser 4 bits de CDCE, ce qui permet d'avoir une détection/correction de 1 erreur par mot qui permet de se protéger par exemple des cas d'altération de bit par rayonnement particulaire alpha. Dans ce cas, sur les 16 lignes du bus de données DATA entre la mémoire 12 et le MSI 18, seules 12 lignes commuteront lors des opérations de lecture/écriture par l'unité centrale 14 ; les 4 autres lignes, maintenues à un niveau logique constant, n'induiront pas de consommation parasite.

On notera que les bits du code opération et les bits du CDCE peuvent être soit séparés, soit entrelacés - pour améliorer encore la protection.

La zone B, quant à elle, est réservée au stockage des données Holter.

Afin d'accroître la capacité, le stockage se fait non par mots de 16 bits comme dans le cas de la zone A, mais par octets, en accédant séparément aux 8 bits de poids fort (UB) ou aux 8 bits de poids faible (LB) de chaque mot mémoire.

L'absence de protection logique des informations stockées en zone B permet de doubler la capacité de stockage, l'espace 24 de la zone B, correspondant aux adresses physiques 10000 à 3FFFF, étant en fait divisé en une première zone 26, d'adresses logiques 10000 à 3FFFF et une seconde zone 28, d'adresses logiques 40000 à 6FFFF. La sélection de l'une ou l'autre zone 26 ou 28 se fait par application au circuit RAM 12 d'un signal de sélection LB (zone 26) ou UB (zone 28) en même temps que le signal de sélection d'écriture/lecture WR.

En accédant ainsi séparément, par le MSI, aux 8 bits de poids faible ou aux 8 bits de poids fort du mot de 16 bits, on peut stocker deux octets d'information classique par mot de 16 bits de la mémoire. Certes, les données n'y bénéficient pas d'une protection (à la différence du code opération stocké en zone A), mais leur perte accidentelle n'est pas critique pour le patient. Néanmoins, on choisira avantageusement un circuit mémoire protégé par une couche polyimide arrêtant les particules alpha.

On va maintenant exposer la manière dont le circuit d'interfaçage MSI 18 gère les accès, en lecture ou en écriture, à l'une ou l'autre des zones, A ou B, du circuit RAM de la mémoire 12.

On va tout d'abord décrire les accès en lecture en zone A.

L'unité centrale 14 peut exécuter un code stocké en ROM 16 ou bien en RAM 12. Pour exécuter un code stocké en RAM, l'unité centrale accède à ce code stocké à une adresse particulière de la zone A ; pendant cette opération, la zone A est protégée en écriture par le microcontrôleur, c'est-à-dire que toute tentative d'écriture dans cette zone provoquerait une ré-initialisation du système.

Le circuit MSI exécute les opérations suivantes :
- tout d'abord, le MSI accède parallèlement, à l'adresse désignée de la zone A, au code opération (CO, 8 bits) et au code détecteur et correcteur d'erreurs (CDCE, n = 1 à 8 bits) ;
- le MSI sépare les 8 bits de CO des n bits de CDCE, avec désentrelacement de ces bits si nécessaire ;
- le MSI recalcule les bits de CDCE à partir des 8 bits de CO ;
- le MSI compare les bits de CDCE calculés aux bits de CDCE lus dans la mémoire ;
- en cas de concordance, alors le MSI transmet au CPU 14 les 8 bits de CO;
- en cas de non-concordance, le MSI corrige, si cela est possible, l'erreur à partir du CDCE lu et transmet en temps réel au CPU le CO corrigé. Une interruption IT est générée pour le CPU, qui récupère sur cette interruption l'adresse du code où l'erreur a été détectée ainsi que la donnée corrigée, ces paramètres étant fournis par le MSI. Une partie ou la totalité de la zone A est alors déprotégée en écriture, permettant ainsi une écriture du CO corrigé à l'adresse considérée. Le CO corrigé est réécrit à l'adresse indiquée via le MSI qui automatiquement recalcule le CDCE, l'adjoint (ou l'entrelace) au CO, et stocke physiquement l'ensemble à l'adresse indiquée dans la zone A. On notera que, par raison de sécurité, cette procédure logicielle spécifique de correction et de réécriture est avantageusement conservée dans la ROM 16 du microcontrôleur ;
- enfin, en cas d'erreur détectée mais non correctible, c'est-à-dire généralement d'une pluralité d'erreurs sur un même mot, le MSI génère un indicateur, lu par l'unité centrale 14 qui, dans cette situation, n'exécute plus le code stocké en RAM, mais exécute un code de repli (code de sécurité), stocké dans la ROM 16.

On va maintenant décrire le cas d'un accès en écriture dans la zone A, correspondant au chargement initial, ou à la mise à jour, du code logiciel stocké dans cette zone.

Dans ce cas, le rôle du MSI consiste à :
- calculer les bits de CDCE à partir des bits de CO transmis par l'unité centrale 14 ou le circuit DMA (selon la voie par laquelle transite le programme à télécharger) ;
- former le mot contenant les 8 bits de code et les n bits de CDCE ; et
- écrire ce mot, correspondant au code protégé, dans la zone A de la RAM à l'adresse indiquée.

On va maintenant décrire le cas d'un accès en zone B.

Cet accès en zone B se fait pour des lectures ou écritures de données Holter, qui sont constituées de données élémentaires stockées sur des mots de 8 bits.

Comme on l'a indiqué plus haut, la structure de RAM utilisée permet d'accéder séparément aux 8 bits de poids faible ou aux 8 bits de poids fort de chaque mot de la zone B, par application d'un signal de sélection UB/LB approprié.

Pour que cette organisation particulière de la RAM soit transparent à l'unité centrale 14 et au circuit DMA 20, il faut que les adresses vues de ces circuits (adresses logiques) s'étendent de 10000 à 6FFFF, la zone des bits de poids faible étant adressée entre 10 000 et 3FFFF (adresses toujours vues de l'unité centrale ou du circuit DMA) et la zone des bits de poids fort, entre 40000 et 6FFFF.

À partir des adresses vues de l'unité centrale ou du circuit DMA, le circuit MSI recalcule l'adresse réelle (physique) de la RAM, comprise entre 10000 et 3FFFF, et génère les signaux correspondants UB/LB selon les bits à adresser, de poids faible ou de poids fort. Cette gestion de la mémoire, transparente pour l'unité centrale, simplifie grandement l'écriture du logiciel de gestion des données Holter, puisque cette unité centrale ne "voit" qu'un seul et même espace adressable continu, constitué des adresses 10000 à 6FFFF.

On a décrit l'invention ci-dessus dans le cadre d'un microcontrôleur travaillant avec un code opération sur 8 bits en parallèle, mais le principe de l'invention est bien entendu transposable à un microcontrôleur de plus grande puissance, en choisissant par exemple une mémoire organisée en mots de 32 bits pour un microcontrôleur 16 bits.

## Revendications

1. Un dispositif médical actif implantable ou d'enregistrement de données Holter comportant :
- un microcontrôleur (10) comprenant un microprocesseur (14),
- des moyens d'acquisition de données médicales,
- une première mémoire (22), configurée pour mémoriser un logiciel de pilotage du microprocesseur (14), et
- une deuxième mémoire (24), configurée pour mémoriser les données médicales recueillies par les moyens d'acquisition,
dans lequel la première et la deuxième mémoire sont, respectivement, un premier (22) et un deuxième (24) secteur d'un unique composant mémoire commun (12),
ledit composant mémoire commun (12) est un unique circuit de mémoire vive, soit circuit RAM, et
le microcontrôleur (10) comprend en outre un circuit d'interface (18), coopérant avec le microprocesseur (14) et l'unique composant mémoire commun (12) ;
**caractérisé en ce que**
- le microprocesseur (14) opère sur N bits en parallèle, ledit composant mémoire commun (12) est une mémoire organisée en mots de 2N bits,
- ledit logiciel de pilotage est mémorisé dans des mots de 2N bits du premier secteur (22) avec une protection logique gérée par le circuit d'interface (18), chacun de ces mots comprenant N bits de code opération formant instruction de commande du microprocesseur (14),associés à n bits de code détecteur et correcteur d'erreur, avec 1 ≤ n ≤ N,
- lesdites données médicales sont formées de données élémentaires de N bits mémorisées dans des mots de 2N bits du deuxième secteur (24) sans protection logique, chacun de ces mots comprenant N bits de poids fort et N bits de poids faible mémorisant chacun une donnée médicale élémentaire, et
- le circuit d'interfaçage (18) est configuré, de manière sélective en fonction des dites commandes délivrées par le microprocesseur (14), pour :
• lire ou écrire parallèlement les N+n bits d'un mot du premier secteur (22), ou bien
• lire ou écrire les N bits de poids faible d'un mot du deuxième secteur (24), ou bien
• lire ou écrire les N bits de poids fort d'un mot du deuxième secteur (24).

2. Le dispositif médical actif de la revendication 1, dans lequel le circuit d'interfaçage (18) est en outre configuré, après lecture des N+n bits d'un mot du premier secteur (22), pour :
• analyser les N bits du code opération et les n bits du code détecteur et correcteur d'erreur de manière à vérifier l'intégrité de ce code opération, et
• seulement en cas d'intégrité avérée, transmettre au microprocesseur (14) les N bits du code opération.

3. Le dispositif médical actif de la revendication 2, dans lequel le circuit d'interfaçage (18) est également configuré, en cas d'intégrité non avérée du code opération, pour :
• restaurer à une valeur intègre les N bits du code opération à partir des n bits du code détecteur et correcteur d'erreur, et
• transmettre au microprocesseur (14) les N bits du code opération ainsi restaurés.

4. Le dispositif médical actif de la revendication 3, dans lequel le circuit d'interfaçage (18) est également configuré, en cas d'intégrité non avérée du code opération, pour :
• recalculer les n bits du code détecteur et correcteur d'erreur correspondant aux N bits restaurés du code opération, et
• commander le microprocesseur (14) de manière à réécrire dans le premier secteur (22) les N bits restaurés du code opération et les n bits recalculés correspondants du code détecteur et correcteur d'erreur.

5. Le dispositif médical actif de la revendication 1 :
- comprenant en outre des moyens de mise à jour du logiciel de pilotage du microprocesseur (14), avec des moyens de téléchargement de séquences de mots de N bits d'instructions de commande du microprocesseur,
- et dans lequel le circuit d'interfaçage est également configuré, pour chaque mot de N bits téléchargé, pour :
• calculer les n bits du code détecteur et correcteur d'erreur correspondant à ces N bits, et
• écrire dans le premier secteur ces N bits et les n bits calculés correspondants du code détecteur et correcteur d'erreur.

6. Le dispositif médical actif de la revendication 4 ou de la revendication 5, comprenant en outre une mémoire morte (16), distincte dudit composant mémoire commun (12), contenant des instructions de pilotage du circuit d'interfaçage pour lesdites opérations de recalcul et de réécriture.

7. Le dispositif médical actif de la revendication 1, dans lequel :
- dans chaque mot du premier secteur (22), les N bits de code opération et les n bits associés de code détecteur et correcteur d'erreur sont mémorisés avec entrelacement mutuel, et
- le circuit d'interfaçage (18) est également configuré, après lecture dans le premier secteur, pour désentrelacer les N+n bits d'un mot en N bits de code opération et n bits associés de code détecteur et correcteur d'erreur.

8. Le dispositif médical actif des revendications 4 et 7 prises en combinaison, ou des revendications 5 et 7 prises en combinaison, dans lequel :
- le circuit d'interfaçage (18) est également configuré pour entrelacer les N bits restaurés du code opération et les n bits recalculés correspondants du code détecteur et correcteur d'erreur avant leur réécriture dans le premier secteur (22).

9. Le dispositif médical actif de la revendication 1 :
- comprenant en outre un circuit d'accès direct mémoire (20) coopérant avec le circuit d'interfaçage (18),
- et dans lequel le circuit d'interfaçage (18) est également configuré, de manière sélective en fonction de commandes délivrées par le circuit d'accès direct mémoire (20), pour :
• lire ou écrire les N bits de poids faible d'un mot du deuxième secteur (24), ou bien
• lire ou écrire les N bits de poids fort d'un mot du deuxième secteur (24), ou bien
• lire ou écrire les N+n bits du premier secteur (22).

10. Le dispositif médical actif de la revendication 1, dans lequel ladite première mémoire (22) est intégrée au même circuit que celui du microprocesseur (14).

## Patentansprüche

1. Implantierbare aktive medizinische Vorrichtung zur Aufzeichnung von Holter-Daten, umfassend:
- einen Mikrokontroller (10), umfassend einen Mikroprozessor (14),
- Mittel zur Erfassung medizinischer Daten,
- einen ersten Speicher (22), der eingerichtet ist, um eine Steuersoftware des Mikroprozessors (14) zu speichern, und
- einen zweiten Speicher (24), der eingerichtet ist, um die von den Erfassungsmitteln erfassten medizinischen Daten zu speichern,
wobei der erste und der zweite Speicher ein erster (22) bzw. ein zweiter (24) Sektor einer einzigen gemeinsamen Speicherkomponente (12) sind,
wobei die gemeinsame Speicherkomponente (12) eine einzige Arbeitsspeicherschaltung, d.h. RAM-Schaltung, ist,
und
wobei der Mikrokontroller (10) ferner eine Schnittstellenschaltung (18) umfasst, die mit dem Mikroprozessor (14) und der einzigen gemeinsamen Speicherkomponente (12) zusammenwirkt,
**dadurch gekennzeichnet, dass**
- der Mikroprozessor (14) auf N Bits parallel arbeitet, wobei die gemeinsame Speicherkomponente (12) ein in Worten zu 2N Bits organisierter Speicher ist,
- die Steuersoftware in Worten von 2N Bits des ersten Sektors (22) mit einem logischen Schutz, der von der Schnittstellenschaltung (18) verwaltet wird, gespeichert ist, wobei jedes dieser Worte N Betriebscodebits umfasst, die eine Steueranweisung des Mikroprozessors (14) bilden und n Erfassungscode- und Fehlerkorrekturbist zugeordnet sind, wobei 1 ≤ n ≤ N,
- wobei die medizinischen Daten von Elementardaten von N Bits, die in Worten von 2N Bits des zweiten Sektors (24) ohne logischen Schutz gespeichert sind, gebildet sind, wobei jedes dieser Worte N Bits mit starkem Gewicht und N Bits mit geringem Gewicht umfasst, die jeweils ein medizinisches Elementardatum speichern, und
- die Schnittstellenschaltung (18) selektiv in Abhängigkeit von den vom Mikroprozessor (14) gelieferten Befehlen eingerichtet ist, um:
• die N+n Bits eines Wortes des ersten Sektors (22) parallel zu lesen oder schreiben, oder auch
• die N Bits mit geringem Gewicht eines Wortes des ersten Sektors (24) zu lesen oder zu schreiben, oder auch
• die N Bits mit starkem Gewicht eines Wortes des zweiten Sektors (24) zu lesen oder zu schreiben.

2. Aktive medizinische Vorrichtung nach Anspruch 1, bei der die Schnittstellenschaltung (18) ferner eingerichtet ist, um nach dem Lesen der N+n Bits eins Wortes des ersten Sektors (22):
• die N Betriebscodebits und die n Erfassungscode- und Fehlerkorrekturbits zu analysieren, um die Integrität dieses Betriebscodes zu überprüfen, und
• nur im Falle einer vorhandenen Integrität an den Mikroprozessor (14) die N Betriebscodebits zu übertragen.

3. Aktive medizinische Vorrichtung nach Anspruch 2, bei der die Schnittstellenschaltung (18) auch eingerichtet ist, um im Falle einer nicht vorhandenen Integrität des Betriebscodes:
• die N Betriebscodebits auf Basis der n Erfassungscode- und Fehlerkorrekturbits auf einem integren Wert wiederherzustellen, und
• an den Mikroprozessor (14) die so wiederhergestellten N Betriebscodebits zu übertragen.

4. Aktive medizinische Vorrichtung nach Anspruch 3, bei der die Schnittstellenschaltung (18) auch eingerichtet ist, um im Falle einer nicht vorhandenen Integrität des Betriebscodes:
• die n Erfassungscode- und Fehlerkorrekturbits entsprechend den N wiederhergestellten Betriebscodebits neu zu berechnen, und
• den Mikroprozessor (14) zu steuern, um in den ersten Sektor (22) wieder die N wiederhergestellten Betriebscodebits und die entsprechenden n neu berechneten Erfassungscode- und Fehlerkorrekturbits zu schreiben.

5. Aktive medizinische Vorrichtung nach Anspruch 1:
- ferner umfassend Mittel zur Aktualisierung der Steuersoftware des Mikroprozessors (14) mit Mitteln zum Herunterladen von Wortfolgen von N Steueranweisungsbits des Mikroprozessors,
- und bei der die Schnittstellenschaltung auch eingerichtet ist, um für jedes heruntergeladene Wort von N Bits:
• die n Erfassungscode- und Fehlerkorrekturbits entsprechend diesen N Bits zu berechnen, und
• in den ersten Sektor diese N Bits und die entsprechenden n berechneten Erfassungscode- und Fehlerkorrekturbits zu schreiben.

6. Aktive medizinische Vorrichtung nach Anspruch 4 oder Anspruch 5, ferner umfassend einen Festspeicher (16), der sich von der gemeinsamen Speicherkomponente (12) unterscheidet und Steueranweisungen der Schnittstellenschaltung für die Vorgänge des Neuberechnens und Neuschreibens enthält.

7. Aktive medizinische Vorrichtung nach Anspruch 1, bei der:
- in jedem Wort des ersten Sektors (22) die N Betriebscodebits und die zugehörigen n Erfassungscode- und Fehlerkorrekturbits mit wechselseitiger Verflechtung gespeichert sind, und
- die Schnittstellenschaltung (18) auch eingerichtet ist, um nach dem Lesen im ersten Sektor die N+n Bits eines Wortes in N Betriebscodebits und n zugehörige Erfassungscode- und Fehlerkorrekturbits zu entflechten.

8. Aktive medizinische Vorrichtung nach den Ansprüchen 4 und 7 in Kombination oder nach den Ansprüchen 5 und 7 in Kombination, bei der:
- die Schnittstellenschaltung (18) auch eingerichtet ist, um die N wiederhergestellten Betriebscodebits und die entsprechenden n neu berechneten Erfassungscode- und Fehlerkorrekturbits vor ihrem Wiedereinschreiben in den ersten Sektor (22) zu verflechten.

9. Aktive medizinische Vorrichtung nach Anspruch 1:
- ferner umfassend eine direkte Speicherzugriffsschaltung (20), die mit der Schnittstellenschaltung (18) zusammenwirkt,
- und bei der die Schnittstellenschaltung (18) auch eingerichtet ist, um selektiv in Abhängigkeit von von der direkten Speicherzugriffsschaltung (20) gelieferten Befehlen:
• die N Bits mit geringem Gewicht eines Wortes des zweiten Sektors (24) zu lesen oder zu schreiben, oder auch
• die N Bits mit starkem Gewicht eines Wortes des zweiten Sektors (24) zu lesen oder zu schreiben, oder auch
• die N+n Bits des ersten Sektors (22) zu lesen oder zu schreiben.

10. Aktive medizinische Vorrichtung nach Anspruch 1, bei der der erste Speicher (22) in dieselbe Schaltung wie jene des Mikroprozessors (14) integriert ist.

## Claims

1. An implantable active medical device or device for recording Holter data comprising:
- a microcontroller (10) comprising a microprocessor (14),
- means for acquiring medical data,
- a first memory (22), configured to store software for driving the microprocessor (14), and
- a second memory (24), configured to store the medical data gathered by the acquisition means,
in which the first and the second memory are, respectively, a first (22) and a second (24) sector of a single common memory component (12),
the said common memory component (12) is a single random-access memory circuit, i.e. RAM circuit,
and
the microcontroller (10) furthermore comprises an interface circuit (18), cooperating with the microprocessor (14) and the single common memory component (12);
**characterized in that**
- the microprocessor (14) operates on N bits in parallel, the said common memory component (12) is a memory organized as words of 2N bits,
- the said driving software is stored in words of 2N bits of the first sector (22) with logic protection managed by the interface circuit (18), each of these words comprising N bits of operation code forming control instruction for the microprocessor (14), and which are associated with n bits of error detector and corrector code, with 1 ≤ n ≤ N,
- the said medical data are formed of N-bit elementary data stored in words of 2N bits of the second sector (24) without logic protection, each of these words comprising N high-order bits and N low-order bits each storing an elementary medical datum, and
- the interfacing circuit (18) is configured, in a selective manner as a function of the said commands delivered by the microprocessor (14), to:
• read or write in parallel the N+n bits of a word of the first sector (22), or else
• read or write the N low-order bits of a word of the second sector (24), or else
• read or write the N high-order bits of a word of the second sector (24).

2. The active medical device of Claim 1, in which the interfacing circuit (18) is furthermore configured, after reading of the N+n bits of a word of the first sector (22), to:
• analyse the N bits of the operation code and the n bits of the error detector and corrector code so as to verify the integrity of this operation code, and
• only in case of substantiated integrity, transmit the N bits of the operation code to the microprocessor (14).

3. The active medical device of Claim 2, in which the interfacing circuit (18) is also configured, in case of unsubstantiated integrity of the operation code, to:
• restore to a dependable value the N bits of the operation code on the basis of the n bits of the error detector and corrector code, and
• transmit the N bits thus restored of the operation code to the microprocessor (14).

4. The active medical device of Claim 3, in which the interfacing circuit (18) is also configured, in case of unsubstantiated integrity of the operation code, to:
• recalculate the n bits of the error detector and corrector code corresponding to the N restored bits of the operation code, and
• control the microprocessor (14) in such a way as to rewrite in the first sector (22) the N restored bits of the operation code and the corresponding n recalculated bits of the error detector and corrector code.

5. The active medical device of Claim 1:
- furthermore comprising means for updating the software for driving the microprocessor (14), with means for downloading sequences of words of N bits of control instructions for the microprocessor,
- and in which the interfacing circuit is also configured, for each downloaded word of N bits, to:
• calculate the n bits of the error detector and corrector code corresponding to these N bits, and
• write in the first sector these N bits and the corresponding n calculated bits of the error detector and corrector code.

6. The active medical device of Claim 4 or of Claim 5, furthermore comprising a read-only memory (16), distinct from the said common memory component (12), containing instructions for driving the interfacing circuit for the said recalculation and rewriting operations.

7. The active medical device of Claim 1, in which:
- in each word of the first sector (22), the N bits of operation code and the n associated bits of error detector and corrector code are stored with mutual interleaving, and
- the interfacing circuit (18) is also configured, after reading in the first sector, to deinterleave the N+n bits of a word into N bits of operation code and n associated bits of error detector and corrector code.

8. The active medical device of Claims 4 and 7 taken in combination, or of Claims 5 and 7 taken in combination, in which:
- the interfacing circuit (18) is also configured to interleave the N restored bits of the operation code and the corresponding n recalculated bits of the error detector and corrector code before their rewriting in the first sector (22).

9. The active medical device of Claim 1:
- furthermore comprising a memory direct access circuit (20) cooperating with the interfacing circuit (18),
- and in which the interfacing circuit (18) is also configured, in a selective manner as a function of commands delivered by the memory direct access circuit (20), to:
• read or write the N low-order bits of a word of the second sector (24), or else
• read or write the N high-order bits of a word of the second sector (24), or else
• read or write the N+n bits of the first sector (22).

10. The active medical device of Claim 1, in which the said first memory (22) is integrated into the same circuit as that of the microprocessor (14).
